# EUROPEAN PATENT APPLICATION

(11) **EP 4 470 577 A1**
(43) Date of publication of application: **04.12.2024**
(21) Application number: 23747328.5
(22) Date of filing: 26.01.2023
(51) Int. Cl.: A61M 1/36, A61M 25/00

(54) **ARTERIAL CANNULA AND CANNULA SET FOR ECMO COMPRISING SAME**

(30) Priority: 27.01.2022 KR 20220012550
(71) Applicant: CHUNGNAM NATIONAL UNIVERSITY HOSPITAL, Daejeon 35015 (KR)
(72) Inventor: CHOI, Young Kyun, Sejong 30092 (KR)
(74) Representative: Müller Schupfner & Partner Patent- und Rechtsanwaltspartnerschaft mbB (Hamburg)
(86) International application number: PCT/KR2023/001164
(87) International publication number: WO 2023/146287

(57) **Abstract**

The present invention relates to an arterial cannula and an ECMO cannula set comprising same, and more particularly, to an arterial cannula having two or more blood flows in a single catheter and an ECMO cannula set comprising same. The arterial cannula according to one embodiment of the present invention may comprise: a catheter that is inserted into the body; a graspable main body in fluid communication with the catheter; and an auxiliary lumen having an outlet on the outer surface of the catheter and an inlet on the outer surface of the main body. The present invention has the advantage that blood flow to the distal parts can be generated using the structure of an existing arterial cannula.

## Description

### Technical Field

The present disclosure relates to an arterial cannula and a cannula set for ECMO including the same, and more particularly, to an arterial cannula having two or more blood flows and a cannula set for ECMO including the same.

### Background Art

An extra-corporeal membrane oxygenation device, that is, an ECMO device, refers to a device for assisting the circulation function of a patient when the cardiorespiratory function of the patient is not normal.

That is, the ECMO device can filter out carbon dioxide included in blood and assist a blood circulation by supplying oxygen. Accordingly, ECMO therapy is often performed on an emergency basis with respect to a cardiac arrest patient or a patient with heart failure that results from a coronary artery disease.

The ECMO therapy includes veno-venous (W)-ECMO therapy in which venous blood is extracted from a vein of a patient and then supplied to the vein of the patient again after the supply of oxygen and the exchange of gases by using the ECMO device and veno-arterial (VA)-ECMO therapy in which venous blood is extracted from a vein of a patient and then supplied to an artery of the patient again after the supply of oxygen and the exchange of gases by using the ECMO device.

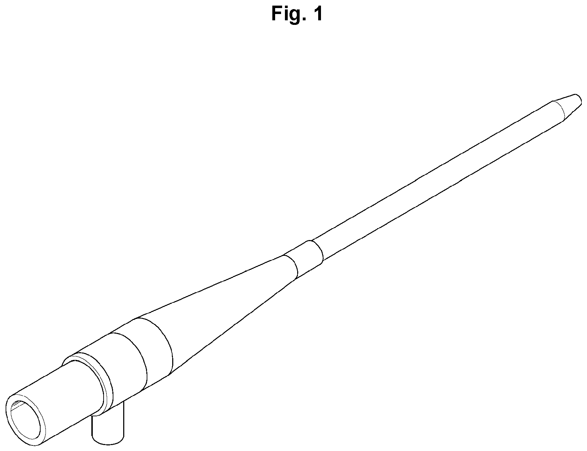

In conventional ECMO therapy, only one blood flow may be formed in the body because an arterial cannula, such as that illustrated in FIG. 1, is used. Accordingly, in particular, in the case of a patient on which the VA-ECMO therapy is being performed, complications, such as that legs are necrotic, may occur because a blood flow is not well circulated from the insertion part of an arterial cannula (i.e., an A-line cannula) to a distal part, such as a hand or foot of the patient.

In order to prevent complications attributable to the execution of the VA-ECMO therapy, a surgical procedure of forming a separate blood flow in a distal part by inserting a distal catheter is additionally performed. However, in general, such a distal catheter insertion surgical procedure has a problem in that the execution of distal perfusion is not easy because the surgical procedure is performed after an A-line cannula is inserted and thus a blood flow is already reduced at a portion on which the surgical procedure is to be performed.

Accordingly, there may be a need for an ECMO catheter capable of preventing the occurrence of complications even without an additional surgical procedure (e.g., distal perfusion) for a patent on which the VA-ECMO therapy is performed.

As a prior art related to the ECMO catheter, Korean Patent Application Publication No. 10-2018-0089150 (August 8, 2018, ECMO catheter) has disclosed an ECMO catheter capable of easily adjusting a location at which blood is sucked and a location from which blood is discharged, in order to solve problems in that it is difficult to adjust a location from which blood to which oxygen is supplied is discharged and it is difficult to adjust a distance from a tricuspid valve, when two tubes are overlapped and it is difficult to adjust the location of a hole.

### DISCLOSURE

### Technical Problem

The present disclosure has been contrived to solve the aforementioned problems, and an object of the present disclosure is to provide an arterial cannula capable of preventing complications which may occur in a patient on which VA-ECMO therapy has been performed without an additional surgical procedure by improving the structure of an arterial cannula, and a cannula set for ECMO including the same.

Objects of the present disclosure are not limited to the aforementioned object, and the other objects not described above may be evidently understood from the following description by those skilled in the art.

### Technical Solution

As an embodiment of the present disclosure, there is provided an artery catheter.

The arterial cannula according to an embodiment of the present disclosure may include a catheter inserted into a body and having a tube form, a main body being in fluid communication with the catheter and having a graspable tube form, and an auxiliary lumen having an exit provided in an outer surface of the catheter and an inlet provided in an outer surface of the main body.

In the arterial cannula according to an embodiment of the present disclosure, the auxiliary lumen may be formed in an outer wall of the catheter and the main body and may be separated from a main lumen that is provided at the center of the catheter and the main body.

In the arterial cannula according to an embodiment of the present disclosure, the auxiliary lumen may have a U shape.

In the arterial cannula according to an embodiment of the present disclosure, the auxiliary lumen may be provided in a plural number in the length direction of the main body and the catheter so that the plurality of auxiliary lumens is spaced apart from each other at predetermined intervals.

As an embodiment of the present disclosure, there is provided a cannula set for ECMO.

The cannula set for ECMO according to an embodiment of the present disclosure may include the aforementioned any one artery cannula and a distal catheter that is inserted into the inlet provided in the main body and that is inserted into the body through the exit provided in the catheter.

### Advantageous Effects

According to the present disclosure, there is an advantage in that a flow of blood to a distal part can be formed by using the structure of the existing arterial cannula.

Furthermore, according to the present disclosure, there is an advantage in that complications attributable to the execution of VA-ECMO therapy can be prevented without an additional surgical procedure, such as distal perfusion.

Effects of the present disclosure which may be obtained in the present disclosure are not limited to the aforementioned effects, and the other effects not described above may be evidently understood by a person having ordinary knowledge in the art to which the present disclosure pertains from the following description.

### Brief Description of Drawings

FIG. 1 is a perspective view of a conventional arterial cannula.
FIG. 2a and 2b are a perspective view and plan view of an arterial cannula according to an embodiment of the present disclosure.
FIG. 3a and 3b are a cross-sectional view of the arterial cannula according to an embodiment of the present disclosure.
FIG. 4a and 4b are a perspective view and plan view of an arterial cannula according to another embodiment of the present disclosure.
FIG. 5 is an enlarged view illustrating a form in which a cannula set for ECMO according to an embodiment of the present disclosure has been inserted.
FIG. 6 is an enlarged view illustrating a form in which a cannula set for ECMO according to another embodiment of the present disclosure has been inserted.

### Best Mode

As an embodiment of the present disclosure, there is provided an artery catheter.

The arterial cannula according to an embodiment of the present disclosure may include a catheter inserted into a body and having a tube form, a main body being in fluid communication with the catheter and having a graspable tube form, and an auxiliary lumen having an exit provided in an outer surface of the catheter and an inlet provided in an outer surface of the main body.

In the arterial cannula according to an embodiment of the present disclosure, the auxiliary lumen may be formed in an outer wall of the catheter and the main body and may be separated from a main lumen that is provided at the center of the catheter and the main body.

In the arterial cannula according to an embodiment of the present disclosure, the auxiliary lumen may have a U shape.

In the arterial cannula according to an embodiment of the present disclosure, the auxiliary lumen may be provided in a plural number in the length direction of the main body and the catheter so that the plurality of auxiliary lumens is spaced apart from each other at predetermined intervals.

As an embodiment of the present disclosure, there is provided a cannula set for ECMO.

The cannula set for ECMO according to an embodiment of the present disclosure may include the aforementioned any one artery cannula and a distal catheter that is inserted into the inlet provided in the main body and that is inserted into the body through the exit provided in the catheter.

### Mode for Invention

Hereinafter, embodiments of the present disclosure are described in detail with reference to the accompanying drawings so that a person having ordinary knowledge in the art to which the present disclosure pertains may easily practice the embodiments. However, the present disclosure may be implemented in various different forms and is not limited to the embodiments described herein. In the drawings, in order to clarify a description of the present disclosure, parts not related to the description are omitted, and similar reference numbers are used to refer to similar parts throughout the specification.

Terms used in this specification are briefly described, and embodiments of the present disclosure are then described in detail.

Terms used in the present disclosure are common terms which are now widely used by taking into consideration functions in the present disclosure, but the terms may be changed depending on an intention of a technician skilled in the art, a precedent, or the advent of a new technology. Furthermore, in a specific case, some terms are randomly selected by the applicant. In this case, the meaning of a corresponding term will be described in detail in a descriptive part of a corresponding invention. Accordingly, terms used in the present disclosure should not be simply defined based on their names, but should be defined based on their substantial meanings and contents over the present disclosure.

In the entire specification, unless explicitly described to the contrary, the word "include" and variations, such as "includes" or "including", will be understood to imply the inclusion of stated components but not the exclusion of any other components. Furthermore, the term "... unit" or "module" described in the specification means a unit for processing at least one function or operation, and the unit may be implemented by hardware or software or a combination of hardware and software. Furthermore, throughout this specification, when it is described that one component is "connected" to the other component, the one component may be "directly connected" to the other component or may be "indirectly connected" to the other component through a third component.

Hereinafter, the present disclosure is described in detail with reference to the accompanying drawings.

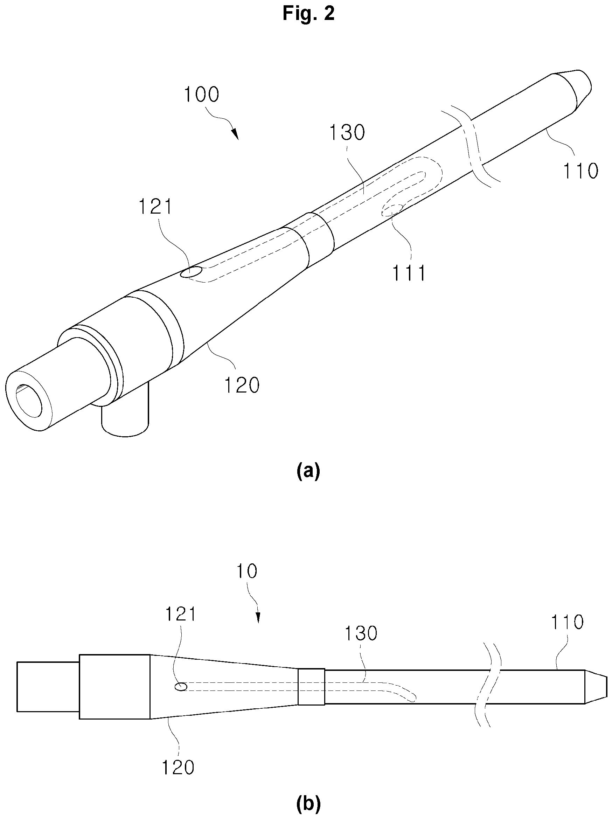

FIG. 2(a) is a perspective view of an arterial cannula 100 according to an embodiment of the present disclosure. FIG. 2(b) is a plan view of the arterial cannula 100 according to an embodiment of the present disclosure.

Referring to FIG. 2(a) and 2(b), the arterial cannula 100 according to an embodiment of the present disclosure includes a catheter 110, a main body 120, and an auxiliary lumen 130.

The catheter 110 is a part that is inserted into the body. The catheter 110 is inserted into an artery, and performs a role as a passage through which blood on which the supply of oxygen and the exchange of gases have been completed is supplied to an artery through an ECMO device.

According to an embodiment, the catheter 110 may be formed in a tube form made of a plastic material, such as polyvinyl chloride (PVC), or a rubber material, and may be formed of synthetic resin made of a transparent material, for example.

The main body 120 is in fluid communication with the catheter 110, and is a graspable part. For example, the main body 120 may be a part which may be grasped by an operator in a surgical procedure for inserting the arterial cannula 100 into the body or a surgical procedure for inserting a distal catheter 200.

According to an embodiment, the main body 120 may be made of a plastic material, such as polyvinyl chloride (PVC), or a rubber material, and may be formed in the form of a tube having a shape in which the cross section of the tube is reduced toward the catheter 110.

The auxiliary lumen 130 may be formed so that an inlet 121 is provided in an outer surface of the main body 120 and an exit 111 is provided in an outer surface of the catheter 110. In this case, it will be preferred that the auxiliary lumen 130 is not in fluid communication with a main lumen 140 that is provided at the center of the catheter 110 and the main body 120.

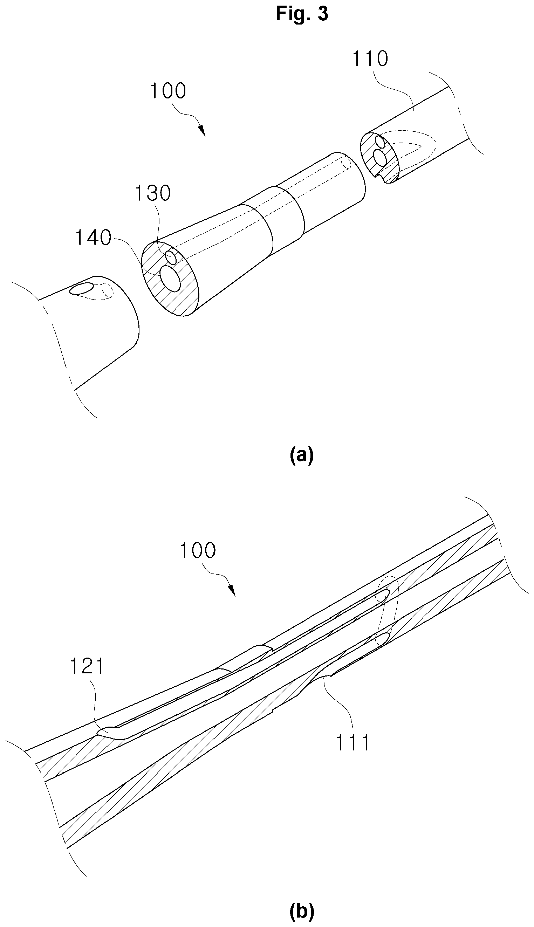

The auxiliary lumen 130 according to an embodiment of the present disclosure is described in detail with reference to FIG. 3(a) and 3(b).

FIG. 3(a) and 3(b) are a cross-sectional view of the arterial cannula 100 according to an embodiment of the present disclosure.

Referring to FIG. 3(a) and 3(b), in the arterial cannula 100 according to an embodiment of the present disclosure, the auxiliary lumen 130 may be formed in the outer wall of the catheter 110 and the main body 120, and may be separated from the main lumen that is formed at the center of the catheter 110 and the main body 120.

Furthermore, the auxiliary lumen 130 may have a U shape.

That is, the arterial cannula 100 according to an embodiment of the present disclosure has two separated spaces that are divided into the main lumen 140 and the auxiliary lumen 130.

According to an embodiment, the main lumen 140 of the arterial cannula 100 may become a passage through which blood on which the supply of oxygen has been completed is supplied to an artery of a patient through an ECMO device. A separate blood flow to a distal part of a patient may be formed through the distal catheter 200 that is inserted through the auxiliary lumen 130 of the arterial cannula 100.

Furthermore, according to an embodiment of the present disclosure, it will be preferred that the inlet 121 and exit 111 of the auxiliary lumen 130 are disposed at proper locations by calculating a section in which the distal catheter 200 may be inserted or a degree that the arterial cannula 100 is inserted according to a soft tissue thickness of a patient. The reason for this is that a location of the auxiliary lumen 130 at which the distal catheter 200 is inserted in a direction opposite to the direction of the arterial cannula 100 may be different depending on the soft tissue thickness of the patient.

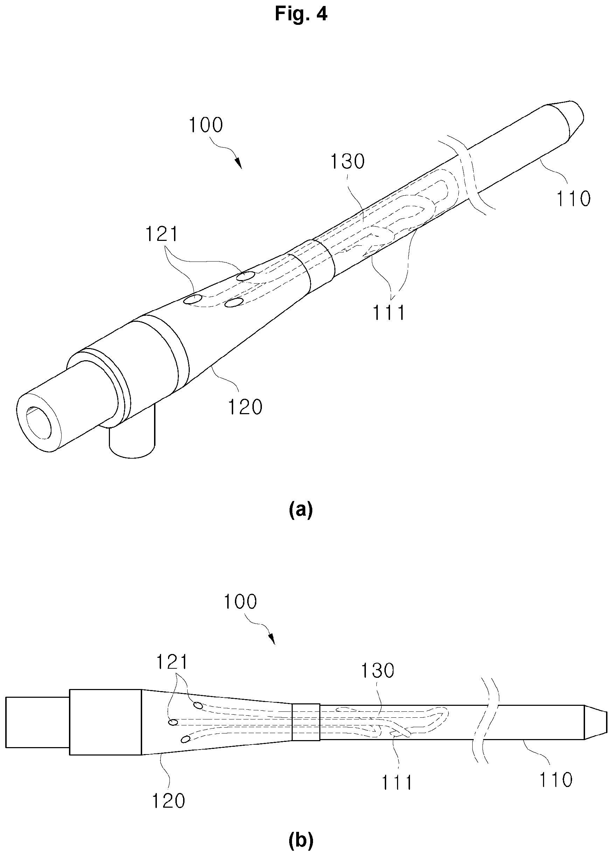

FIG. 4(a) is a perspective view of the arterial cannula 100 according to another embodiment of the present disclosure. FIG. 4(b) is a plan view of the arterial cannula 100 according to another embodiment of the present disclosure.

Referring to FIG. 4(a) and 4(b), in the arterial cannula 100 according to another embodiment of the present disclosure, the auxiliary lumen 130 may be provided in a plural number in the length direction of the main body 120 and the catheter 110 so that the plurality of auxiliary lumens is spaced apart from each other at predetermined intervals.

That is, according to the arterial cannula 100 according to another embodiment of the present disclosure, the distal catheter 200 may be inserted in a direction opposite to the direction of the arterial cannula 100 by selecting any one of the plurality of auxiliary lumens 130 formed in the arterial cannula 100, without calculating a section in which the distal catheter 200 may be inserted or a degree that the arterial cannula 100 is inserted for each soft tissue thickness of a patient.

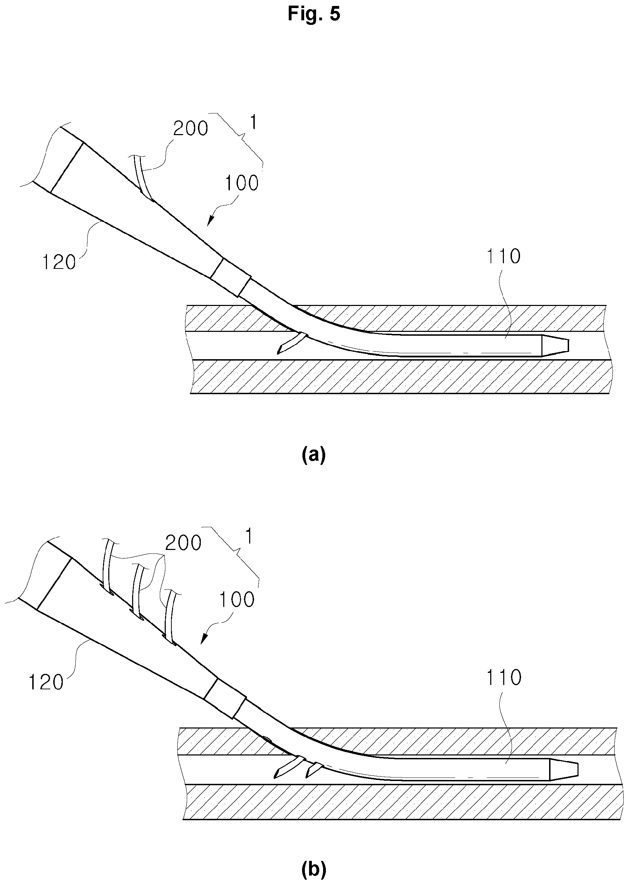

FIG. 5 is an enlarged view illustrating a form in which a cannula set 1 for ECMO according to an embodiment of the present disclosure has been inserted. FIG. 6 is an enlarged view illustrating a form in which the cannula set 1 for ECMO according to another embodiment of the present disclosure has been inserted.

Referring to FIGS. 5 and 6, the cannula set 1 for ECMO according to an embodiment of the present disclosure may include any one of the aforementioned arterial cannulas 100 and the distal catheter 200 that is inserted into the inlet 121 provided in the main body 120 and that is inserted into the body through the exit 111 provided in the catheter 110.

VA-ECMO therapy using the cannula set 1 for ECMO according to embodiments of the present disclosure may be performed through step S100 of inserting the arterial cannula 100 into an artery of a patient, step S200 of inserting a syringe into the inlet 121 of the auxiliary lumen 130, which is provided in the main body 120 of the arterial cannula 100, step S300 of checking that blood flows backward by applying negative pressure to the auxiliary lumen 130 by using the syringe, step S400 of passing a guide wire through the auxiliary lumen 130 through the inlet 121 of the auxiliary lumen 130, step S500 of checking whether the guide wire has been placed within a blood vessel when the length of the guide wire inserted into the auxiliary lumen 130 is longer than the length of the auxiliary lumen 130, and step S600 of inserting the distal catheter 200 into the auxiliary lumen 130 by using the guide wire.

The description of the present disclosure is illustrative, and a person having ordinary knowledge in the art to which the present disclosure pertains will understand that the present disclosure may be easily modified in other detailed forms without changing the technical spirit or essential characteristic of the present disclosure. Accordingly, it should be construed that the aforementioned embodiments are only illustrative in all aspects, and are not limitative. For example, components described in the singular form may be carried out in a distributed form. Likewise, components described in a distributed form may also be carried out in a combined form.

The scope of the present disclosure is defined by the appended claims rather than by the detailed description, and all changes or modifications derived from the meanings and scope of the claims and equivalents thereto should be interpreted as being included in the scope of the present disclosure.

## Claims

1. An artery cannula comprising:
a catheter inserted into a body and having a tube form;
a main body being in fluid communication with the catheter and having a graspable tube form; and
an auxiliary lumen having an exit provided in an outer surface of the catheter and
an inlet provided in an outer surface of the main body.

2. The artery cannula of claim 1, wherein the auxiliary lumen is formed in an outer wall of the catheter and the main body and is separated from a main lumen that is provided at a center of the catheter and the main body.

3. The artery cannula of claim 1, wherein the auxiliary lumen has a U shape.

4. The artery cannula of claim 1, wherein the auxiliary lumen is provided in a plural number in a length direction of the main body and the catheter so that the plurality of auxiliary lumens is spaced apart from each other at predetermined intervals.

5. A cannula set for ECMO, comprising:
the artery cannula according to any one of claims 1 to 4; and
a distal catheter that is inserted into the inlet provided in the main body and that is inserted into the body through the exit provided in the catheter.
